# EUROPEAN PATENT APPLICATION

(11) **EP 2 735 616 A1**
(43) Date of publication of application: **28.05.2014**
(21) Application number: 12802461.9
(22) Date of filing: 12.06.2012
(51) Int. Cl.: C12P 21/02, A23L 1/305, C07K 7/08, C07K 14/47, C12N 1/20, A61K 38/00, A61P 25/28

(54) **PRODUCTION METHOD FOR CASEIN-DERIVED PEPTIDES BY LACTIC ACID FERMENTATION**

(30) Priority: 24.06.2011 JP 2011140737
(71) Applicant: Calpis Co., Ltd., Tokyo 150-0022 (JP)
(72) Inventor: UCHIDA Naoto, Sagamihara-shi Kanagawa 252-0206 (JP); GOTO Hiroaki, Sagamihara-shi Kanagawa 252-0206 (JP); OHSAWA Kazuhito, Sagamihara-shi Kanagawa 252-0206 (JP); OHKI Kohji, Sagamihara-shi Kanagawa 252-0206 (JP)
(74) Representative: Dempster, Robert Charles
(86) International application number: PCT/JP2012/064998
(87) International publication number: WO 2012/176659

(57) **Abstract**

The present invention provides a simple and efficient method for preparing safer compounds which prevent and ameliorate conditions and diseases caused by reduction in brain function and are suitable for use in medicines and food products. Specifically, the present invention provides: a method for preparing peptides each consisting of an amino acid sequence shown in any of SEQ ID NOs: 1-4 or 16, said method being characterized in that mammalian milk or milk protein is fermented using at least one lactic acid bacterium and/or a processed product thereof, and then the peptides are collected from the resultant fermented milk.

## Description

### TECHNICAL FIELD

The present invention relates to a method for preparing a casein-derived peptide by fermentation of a lactic acid bacterium. The present invention also relates to a method for preparing a composition or a functional food comprising the peptide.

### BACKGROUND ART

Symptoms and diseases caused by decreased brain function include depression, schizophrenia, deliria, and dementias (such as cerebrovascular dementia and Alzheimer disease). Particularly, an increase in dementia patients has become a major social problem with the aging of modem society. The symptoms of dementia depend on individuals. Examples of symptoms observed in common include memory disorder, disorientation, and decreased ability to judge/think. Among dementias, those from which many patients suffer are cerebrovascular dementia and Alzheimer disease. For example, for cerebrovascular dementia, cognition/memory disorder appears because decreased cerebral blood flow damages neuronal cells in the cerebral cortex and the hippocampus. Thus, a drug improving cerebral blood flow and a drug protecting cerebral neurons have been applied in addition to treating underlying diseases including hypertension, diabetes, and hypercholesterolemia which potentially cause cerebrovascular disorder. For Alzheimer disease, whose cause remains to be clearly elucidated, the decreased function of cholinergic nerve is considered to be one of the causes thereof because a decrease in the level of acetylcholine as an intracerebral neurotransmitter is observed in patients with the disease (see, for example, Bartus, R.T. et al., Science, 217: 408-414 (1982)). Therefore, for Alzheimer disease, a therapeutic method is predominant which is intended to prevent the decrease of function of cholinergic nerve by increasing the level of acetylcholine.

Currently, as therapeutic drugs for Alzheimer disease, for example, acetylcholinesterase inhibitors such as donepezil hydrochloride are commercially available. However, acetylcholinesterase inhibitors such as donepezil hydrochloride have a disadvantage that they cannot be administered for a long period of time because of their liver toxicity and strong side effects, and are also expensive.

As a report on a peptide having the effect of improving amnesia, it is reported, for example, that lateral ventricular injection or oral administration of 300 mg/kg of XPLPR (where X is L, I, M, F, or W) has an effect of improving scopolamine-induced amnesia, suggesting the release of acetylcholine through brain C3a receptor as one of mechanisms therefor (JP Patent No. 3898389). Scopolamine is considered to cause a decrease of the function of cholinergic nerve as a muscarinic receptor antagonist, and act as an agent inducing brain dysfunction. So, it has been used for producing a model animal for the development of therapeutic drugs for Alzheimer disease. The effect of preventing and/or improving brain dysfunction can be demonstrated, for example, by a behavioral pharmacological test such as a Y-shaped maze test, an eight-arm maze test, or a passive avoidance test. The effect of improving and/or enhancing brain function can be demonstrated by a similar behavioral pharmacological test using a normal animal. However, each of the peptides is required to be orally administered at a large dose or intraperitoneally administered, intraventricularly injected, or by other delivery route to exhibit the action, and does not have a sufficient effect as an orally ingestable substance.

### PRIOR ART DOCUMENT

### PATENT DOCUMENT

Patent Document 1: Patent No. 3898389

### NON-PATENT DOCUMENT

Non-Patent Document: Science. 217, 408-414 (1982)

### SUMMARY OF THE INVENTION

### PROBLEM TO BE SOLVED BY THE INVENTION

With the progress of aging of the society, there is a strong need for the development of such a compound as to have the effect of preventing and also improving symptoms or diseases caused by decreased brain function, which is a safer compound excellent in application to a pharmaceutical product and food. There is also a need for a method for simply and efficiently producing such a compound.

### MEANS FOR SOLVING THE PROBLEM

As a result of intensive studies for solving the above-described objects, the present inventors have found that a peptide(s) derived from a milk protein casein has a brain function-improving action. The present inventors have now found that such a casein-derived peptide(s) can be simply and efficiently produced by fermentation of lactic acid bacteria. Thus, the finding has been obtained that the fermentation of lactic acid bacteria can be used to produce a particular casein-derived peptide(s) and produce the peptide(s) and a composition having a brain function-improving action, thereby accomplishing the present invention.

Thus, the present invention encompasses the following.
[1] A method for preparing a peptide consisting of the amino acid sequence shown in any of SEQ ID NOS: 1 to 4 or 16, comprising fermenting animal milk or milk protein using at least one lactic acid bacterium and/or a treated product thereof and collecting the peptide from the resultant fermented milk.
[2] The method according to [1], wherein the lactic acid bacterium is at least one bacterium belonging to a genus selected from the group consisting of *Lactobacillus, Streptococcus, Bifidobacterium, Enterococcus, Leuconostoc, Lactococcus, Pediococcus,* and *Weissella.*
[3] The method according to [1] or [2], wherein the lactic acid bacterium is at least one selected from the group consisting of *Lactobacillus helveticus, Lactobacillus delbrueckii* subsp. *bulgaricus, Lactobacillus acidophilus, Lactobacillus casei, Lactobacillus amylovorus, Lactobacillus gasseri, Lactobacillus paracasei, Lactobacillus zeae, Lactobacillus rhamnosus, Lactobacillus reuteri, Lactobacillus crispatus, Lactobacillus gallinarum, Lactobacillus brevis, Lactobacillus fermentum, Lactobacillus plantarum,* and *Lactobacillus johnsonii.*
[4] The method according to [1] or [2], wherein the lactic acid bacterium is *Streptococcus thermophilus.*
[5] The method according to [1] or [2], wherein the lactic acid bacterium is *Lactobacillus helveticus* CM4 strain (Accession Number: FERM BP-6060).
[6] The method according to any of [1] to [5], wherein the peptide is collected 3 to 96 hours after the start of the fermentation.
[7] A method for preparing a composition comprising a peptide consisting of the amino acid sequence shown in any of SEQ ID NOS: 1 to 4 or 16, comprising fermenting animal milk or milk protein using at least one lactic acid bacterium and/or a treated product thereof and formulating the resultant fermented milk or the peptide consisting of the amino acid sequence shown in any of SEQ ID NOS: 1 to 4 or 16 collected from the fermented milk.
[8] A composition comprising a peptide consisting of the amino acid sequence shown in any of SEQ ID NOS: 1 to 4 or 16.
[9] A fermented milk composition, which is obtained by fermenting animal milk or milk protein using at least one lactic acid bacterium and/or a treated product thereof and comprises a peptide consisting of the amino acid sequence shown in any of SEQ ID NOS: 1 to 4 or 16.
[10] The composition according to [9], wherein the composition comprises 0.1 µg/ml or more of the peptide.
[11] A food or drink comprising the composition according to any of [8] to [10] incorporated thereinto.
[12] A supplement comprising the composition according to any of [8] to [10] incorporated thereinto.
[13] A method for preparing a functional food or drink, comprising fermenting animal milk or milk protein using at least one lactic acid bacterium and/or a treated product thereof, and incorporating the resultant fermented milk or a peptide consisting of the amino acid sequence shown in any of SEQ ID NOS: 1 to 4 or 16 obtained therefrom, into a food or drink.
[14] Use of at least one lactic acid bacterium or a treated product thereof for producing a peptide consisting of the amino acid sequence shown in any of SEQ ID NOS: 1 to 4 or 16.

### EFFECT OF THE INVENTION

According to the present invention, a method for preparing a casein-derived peptide is provided. The casein-derived peptide has primarily a brain function-improving action and such a functional peptide can be simply and efficiently prepared by the preparation method of the present invention. According to the present invention, a method for preparing a composition or a functional food containing the peptide is also provided.

### BRIEF DESCRIPTION OF THE DRAWINGS

FIG. 1 is a graph showing the time course of the amount of each peptide in the fermented milk using *Lactobacillus helveticus* CM4 strain.
FIG. 2 is a graph showing an effect of preventing scopolamine-induced amnesia of the peptide of SEQ ID NO: 1. ** indicates p<0.01 relative to the water-administered control group. # indicates p<0.05 relative to the scopolamine control group, and ## indicates p<0.01 relative to the scopolamine control group.
FIG. 3 is a graph showing an effect of preventing scopolamine-induced amnesia of the peptide of SEQ ID NO: 1, 2, 7, or 8. ** indicates p<0.01 relative to the water-administered control group. ## indicates p<0.01 relative to the scopolamine control group.
FIG. 4 is a graph showing an effect of preventing scopolamine-induced amnesia of the peptide of SEQ ID NO: 1 or 10. ** indicates p<0.01 relative to the water-administered control group. # indicates p<0.05 relative to the scopolamine control group.
FIG. 5 is a graph showing a memory-enhancing effect of the peptide of SEQ ID NO: 1. * indicates p<0.05 relative to the water-administered control group.
FIG. 6 is a graph showing an effect of preventing scopolamine-induced amnesia of the peptide of SEQ ID NO: 11. ** indicates p<0.01 relative to the water-administered control group. # indicates p<0.05 relative to the scopolamine control group.
FIG. 7 is a graph showing effects of preventing scopolamine-induced amnesia of the peptides of SEQ ID NOS: 12 to 15. ** indicates p<0.01 relative to the water-administered control group. # indicates p<0.05 relative to the scopolamine control group. † indicates p<0.1 relative to the scopolamine control group.
FIG. 8 is a graph showing an effect of preventing scopolamine-induced amnesia of the peptide of SEQ ID NO: 3. ** indicates p<0.01 relative to the water-administered control group. ## indicates p<0.01 relative to the scopolamine control group, and † indicates p<0.1 relative to the scopolamine control group.
FIG. 9 is a graph showing an effect of preventing scopolamine-induced amnesia of the peptide of SEQ ID NO: 3, 4, 16, or 17. ** indicates p<0.01 relative to the water-administered control group. ## indicates p<0.01 relative to the scopolamine control group, and # indicates p<0.05 relative to the scopolamine control group.

### MODE FOR CARRING OUT THE INVENTION

The present invention will be described below in detail.

The present inventor has found that casein-derived peptides having the following amino acid sequences have a brain function-improving action (see Examples 4 to 11 described hereinbelow):
Xaa-Pro-Pro-Leu-Thr-Gln-Thr-Pro-Val-Val-Val-Pro-Pro-Phe-Leu-Gln-Pro-Glu-Xaa (SEQ ID NO: 5), wherein Xaa at position 1 is absent, or represents Ile or Asn-Ile, and Xaa at position 19 is absent, or represents Val-Met; and
Xaa-Met-His-Gln-Pro-His-Gln-Pro-Leu-Pro-Pro-Thr-Val-Met-Phe-Pro-Pro-Gln-Ser-Val-Leu (SEQ ID NO: 6), wherein Xaa at position 1 is absent, or represents Ser-Trp or Leu-Gln-Ser-Trp.

The particularly preferred peptide consisting of the amino acid sequence shown in SEQ ID NO: 5 is a peptide consisting of the amino acid sequence shown in SEQ ID NO: 1 or 2:
Asn-Ile-Pro-Pro-Leu-Thr-Gln-Thr-Pro-Val-Val-Val-Pro-Pro-Phe-Leu-Gln-Pro-Glu (SEQ ID NO: 1); or
Asn-Ile-Pro-Pro-Leu-Thr-Gln-Thr-Pro-Val-Val-Val-Pro-Pro-Phe-Leu-Gln-Pro-Glu-Val-Met (SEQ ID NO: 2).

The particularly preferred peptide consisting of the amino acid sequence shown in SEQ ID NO: 6 is a peptide consisting of the amino acid sequence shown in SEQ ID NO: 3, 4, or 16:
Ser-Trp-Met-His-Gln-Pro-His-Gln-Pro-Leu-Pro-Pro-Thr-Val-Met-Phe-Pro-Pro-Gln-Ser-Val-Leu (SEQ ID NO: 3);
Leu-Gln-Ser-Trp-Met-His-Gln-Pro-His-Gln-Pro-Leu-Pro-Pro-Thr-Val-Met-Phe-Pro-Pro-Gln-Ser-Val-Leu (SEQ ID NO: 4); or
Met-His-Gln-Pro-His-Gln-Pro-Leu-Pro-Pro-Thr-Val-Met-Phe-Pro-Pro-Gln-Ser-Val-Leu (SEQ ID NO: 16).

The present invention relates to a method for preparing a peptide comprising the amino acid sequence shown in SEQ ID NO: 5 or 6 (particularly any of SEQ ID NOs: 1 to 4 or 16) or consisting of the amino acid sequence by fermentation of lactic acid bacteria. The peptide produced according to the present invention may be a peptide consisting of an amino acid sequence in which 1 or several, preferably 1 to 3, more preferably 1 or 2 amino acids are deleted, substituted, or added in the amino acid sequence shown in SEQ ID NO: 5 or 6, provided that it has a desired action, for example, a brain function-improving action. It may also be a salt of the peptide. In the present invention, peptides having a brain function-improving action or other useful functions, related to SEQ ID NO: 5 or 6 are collectively referred to as "functional peptide(s)". In the context of the present invention, the three letter codes and the one letter codes for amino acids and the code for a peptide shall comply with a general rule well-known to those skilled in the art.

The brain function-improving action as one of their functions can be confirmed using a system according to an evaluation system for therapeutic agents for Alzheimer's disease, using, for example, a Y-shaped maze. Specifically, a muscarinic receptor antagonist agent such as scopolamine can be used in rats or mice to cause a decrease of the function of cholinergic nerve and causing brain dysfunction, thereby inducing amnesia, and a test peptide is administered simultaneously with such agent or the test peptide is administered prior to the administration of such agent to confirm an amnesia-preventing action of the test peptide using the percentage of change in spontaneous alternation behavior to different arms and the total number of entries into the maze as indicators in a test using the Y-shaped maze. Alternatively, the brain function-improving action can be confirmed, for example, by performing a novel object recognition test using rats or mice. Specifically, after administering the test peptide and performing a training trial for causing the animal to memorize two objects in a test using an experimental box, the memory is removed by the lapse of time and one of the two objects is exchanged with a novel one; if the exchanged object is remembered, the exploration time for the novel object is increased, which can be used as an indicator to confirm a memory-enhancing action of the test peptide.

According to the present invention, the functional peptide(s) described above can be prepared by fermenting animal milk or milk protein using a lactic acid bacterium or a treated product thereof.

The lactic acid bacterium which can be used in the present invention is a bacterium capable of producing lactic acid from saccharides via fermentation. Examples thereof include bacteria belonging to the genera *Lactobacillus, Streptococcus, Leuconostoc, Lactococcus, Pediococcus, Enterococcus, Bifidobacterium,* and *Weissella.* According to the present invention, lactic acid bacterial strains known in the art can be used as long as bacterial cells or a treated product of lactic acid bacteria can produce the functional peptide(s) by fermentation. The lactic acid bacterium can be a strain which is capable of producing at least one of the functional peptides described above, and is not required to be a strain which produces all of the functional peptides.

Specific examples of lactic acid bacteria include bacteria belonging to the genus *Lactobacillus* such as *Lactobacillus helveticus, Lactobacillus delbrueckii* subsp. *bulgaricus, Lactobacillus acidophilus, Lactobacillus casei, Lactobacillus amylovorus, Lactobacillus gasseri, Lactobacillus paracasei, Lactobacillus zeae, Lactobacillus rhamnosus, Lactobacillus reuteri, Lactobacillus crispatus, Lactobacillus gallinarum, Lactobacillus brevis, Lactobacillusfermentum, Lactobacillus plantarum,* and *Lactobacillus johnsonii.*

In addition, other specific examples of lactic acid bacteria include bacteria belonging to the genus *Streptococcussuch* as *Streptococcus thermophilus.* Examples of bacteria belonging to the genus *Bifidobacterium* include *Bifidobacterium breve, Bifidobacterium longum, Bifidobacterium pseudolongum, Bifidobacterium animalis, Bifidobacterium adolescentis, Bifidobacterium bifidum, Bifidobacterium lactis, Bifidobacterium catenulatum, Bifidobacterium pseudocatenulatum,* and *Bifidobacterium magnum.* Examples of bacteria belonging to the genus *Enterococcus* include *Enterococcus faecalis, Enterococcus hirae,* and *Enterococcus faecium.* Examples of bacteria belonging to the genus *Leuconostoc* include *Leuconostoc mesenteroides* and *Leuconostoc lactis.* Examples of bacteria belonging to the genus *Lactococcus* include *Lactococcus lactis, Lactococcus plantarum,* and *Lactococcus raffinolactis.* Examples of bacteria belonging to the genus *Pediococcus* include *Pediococcus pentosaceus* and *Pediococcus damnosus.* Examples of bacteria belonging to the genus *Weissella* include *Weissella cibaria, Weissella confusa, Weissella halotolerans, Weissella hellenica, Weissella kandleri, Weissella kimchii, Weissella koreensis, Weissella minor, Weissella paramesenteroides, Weissella soli, Weissella thailandensis,* and *Weissella viridescens.*

It can be determined by a method known in the art, for example, as described in the Examples hereinbelow whether or not a lactic acid bacterium or a treated product thereof to be used has an ability to produce the functional peptide(s). Briefly, the ability can be determined by fermenting milk proteins (for example, animal milk) as a substrate using a lactic acid bacterium or a treated product thereof at 25 to 42 °C, preferably 28 to 37 °C, for 5 to 50 hours, and measuring the amount of the functional peptide(s) contained in fermented milk of the milk proteins so obtained.

According to the present invention, any lactic acid bacteria can be used as long as bacterial cells or a treated product thereof have been evaluated as having an ability to produce the functional peptide(s) by a method such as the above method. A preferable example of a strain of a lactic acid bacterium is *Lactobacillus helveticus* CM4 strain (Accession Number: FERM BP-6060). This strain has been confirmed to have an ability to produce the functional peptides in the Examples described hereinbelow. It can produce several types of the functional peptides, and thus, may be one of preferred strains. The *Lactobacillus helveticus* CM4 strain (Accession Number: FERM BP-6060) is deposited by the present applicant as of August 15, 1997, with the International Patent Organism Depositary (IPOD), National Institute of Technology and Evaluation (NITE) (formerly National Institute of Advanced Industrial Science and Technology (AIST)) (Tsukuba Central 6, 1-1-1 Higashi, Tsukuba, Ibaraki, Japan), which is an international depository authority established under the Budapest Treaty for deposition of patent microorganisms. In addition, several types of lactic acid bacteria (including strains belonging to *Lactobacillus* and *Streptococcus*) have been confirmed to have an ability to produce the functional peptide(s) as described in the Examples hereinbelow, and such strains can also be used in the present invention in addition to the above deposited strain.

Also, mutant strains of the above specific bacterial strains can be used in the present invention as long as they have an ability to produce the functional peptide(s). For example, a mutant strain of the *Lactobacillus helveticus* CM4 strain (Accession Number: FERM BP-6060) may have the ability to produce the functional peptide(s) with high probability, and such mutant strain can also be used in the present invention.

As used herein, "mutant strain" means any strain which has been obtained from a parent strain. Specifically, it means a strain which can be obtained from a parent strain by a spontaneous mutation, or a method which artificially enhances frequency of mutations by mutagenesis with a chemical or physical mutagen, or a specific mutagenesis (e.g., genetic engineering). Microorganisms obtained by such methods are subjected to repeated screenings and separations to grow a useful microorganism, thereby obtaining a mutant strain with a desired property.

For example, a mutant strain derived from *Lactobacillus helveticus* CM4 strain (Accession Number: FERM BP-6060) can be distinguished from other lactic acid bacterium strains based on the molecular size distribution of amplified fragments obtained by using polymerase chain reaction (PCR) based on genomic DNA of a lactic acid bacterium. Briefly, whether a lactic acid bacterium to be tested is a mutant strain derived from *Lactobacillus helveticus* CM4 strain (Accession Number: FERM BP-6060) can be determined by preparing a DNA sample from the lactic acid bacterium, amplifying the DNA sample by a PCR method using primers having characteristic sequences (for example, 16S rDNA nucleotide sequence), and analyzing an electrophoresis pattern of amplified fragments. The method for confirming a mutant strain is not limited to the above method, and a mutant strain can be confirmed by a method known in the art, for example, based on bacteriological characteristics. As a mutant strain which was obtained from *Lactobacillus helveticus* CM4 strain (Accession Number: FERM BP-6060) as a parent strain, *Lactobacillus helveticus* CP3232 strain (Accession Number: FERM BP-11271) is known, and can be used according to the present invention. The *Lactobacillus helveticus* CP3232 strain is deposited by the present applicant under Accession Number FERM BP-11271 as of August 4, 2010, with the International Patent Organism Depositary, National Institute of Technology and Evaluation (NITE) (formerly National Institute of Advanced Industrial Science and Technology (AIST)) (Tsukuba Central 6, 1-1-1 Higashi, Tsukuba, Ibarali, Japan), which is an international depository authority established under the Budapest Treaty for deposition of patent microorganisms.

A strain which can be used in the present invention can be obtained by determining whether or not a mutant strain or a treated product of the mutant strain have an ability to produce the functional peptide(s).

A lactic acid bacterium can be prepared via culture under adequate conditions using a medium conventionally used for culture of lactic acid bacteria. A natural medium or a synthetic medium can be used as a culture medium as long as it contains a carbon source, a nitrogen source, mineral salts, and other components and it enables culture of lactic acid bacteria with efficiency. Those skilled in the art can adequately select a known medium appropriate for a bacterial strain to be used. Examples of a carbon source include lactose, glucose, galactose, and blackstrap molasses. Examples of a nitrogen source include organic nitrogen-containing substances such as casein hydrolysate, whey protein hydrolysate, and soy protein hydrolysate. Examples of the mineral salts include phosphate, sodium, potassium, and magnesium. Examples of an appropriate medium for culture of lactic acid bacteria include an MRS liquid medium, a GAM medium, a BL medium, Briggs Liver Broth, animal milk, skim milk, and milk-derived whey. Preferably, a sterilized milk medium containing skim milk can be used.

In addition, culture of lactic acid bacteria can be performed at 20 °C to 50 °C, preferably 25 °C to 42 °C, and more preferably 28 °C to 37 °C under anaerobic conditions. Temperature conditions can be adjusted using a thermostatic bath, a mantle heater, a jacket, or the like. In addition, the term "anaerobic conditions" used herein refers to a low-oxygen environment in which a lactic acid bacterium can proliferate. For instance, anaerobic conditions can be provided by using an anaerobic chamber, an anaerobic box, an airtight container or bag containing a deoxidizer, or the like, or by simply sealing a culture container. The format of culture includes static culture, shake culture, and tank culture. In addition, the period of culture can be determined to be 3 hours to 96 hours. It is preferable to maintain the pH of the medium at 4.0 to 8.0 in the beginning of culture.

A specific example of preparation of lactic acid bacteria is briefly described below. For instance, when *Lactobacillus helveticus* CM4 strain is used, the lactobacillus is inoculated to a sterilized milk medium (e.g., milk medium containing 9.0% (w/w) reconstituted skim milk) at a concentration of about 3 to 5%, followed by overnight culture at 28 to 37 °C (for about 18 to 28 hours). Preferably, the culture procedures may be repeated.

After culture, the obtained culture product of lactic acid bacteria can be directly used, or it may be further subjected to crude purification via centrifugation and/or solid-liquid separation via filtration, and sterilization, etc. according to need.

In addition, a treated product of a lactic acid bacterium obtained by treating bacterial cells of the lactic acid bacterium may be used as long as it has an ability of interest. Alternatively, a treated product of a lactic acid bacterium may be further subjected to treatment. Examples of such treatment are described below.

A fermented milk can be prepared by fermenting raw milk, skim milk, or soymilk using bacterial cells and/or a treated product of a lactic acid bacterium. For instance, a lactic acid bacterium or a lactic acid bacterium subjected to optional treatment may be inoculated to raw milk, skim milk, or soymilk, followed by fermentation under conditions for lactic acid bacteria (substantially equivalent to the above conditions for culture of lactic acid bacteria) known in the art. The thus obtained fermented milk can be directly used, or it may be subjected to optional treatment such as filtration, sterilization, dilution, and/or concentration.

Bacterial cells and/or a treated product of a lactic acid bacterium can be prepared in the form of suspension or diluted solution by suspension or dilution in an adequate solvent. Examples of a solvent that can be used include water, physiological saline, and phosphate buffer saline (PBS).

A sterilized product can be prepared by sterilization treatment of bacterial cells and/or a treated product of a lactic acid bacterium. In order to subject bacterial cells and/or a treated product of a lactic acid bacterium to sterilization treatment, for example, a known technique such as filtration sterilization, radiation disinfection, superheat disinfection, or pressure disinfection can be used.

A heated product can be prepared by heat treatment of bacterial cells and/or a treated product of a lactic acid bacterium. In order to prepare such heated product, high temperature treatment (for example, at 80 °C to 150 °C) of bacterial cells and/or a treated product of a lactic acid bacterium is performed for a certain period of about 10 minutes to 1 hour (e.g., about 10 to 20 minutes).

A disrupted product or a cell-free extract can be prepared by disrupting, fracturing or grinding bacterial cells and/or a treated product of a lactic acid bacterium. For instance, physical disruption (e.g., agitation or filter filtration), enzymatic lysis treatment, chemical treatment and/or autolysis induction treatment can be performed.

An extract can be obtained via extraction of bacterial cells and/or a treated product of a lactic acid bacterium with the use of an adequate aqueous or organic solvent. An extraction method is not particularly limited as long as it is an extraction method using an aqueous or organic solvent as an extraction solvent. For example, a known method such as a method comprising immersing a lactic acid bacterium or a lactic acid bacterium subjected to optional treatment in an aqueous or organic solvent (e.g., water, methanol, or ethanol), or agitating or refluxing it in the solvent can be used.

In addition, bacterial cells and/or a treated product of a lactic acid bacterium can be processed into the form of a powdery product (powder) or granular product via drying. Drying methods include, but not particularly limited to, spray drying, drum drying, vacuum drying, and lyophilization, which can be used alone or in combination. Upon drying, excipients may be added according to need conventionally.

A lactic acid bacterium used in the present invention may be in the form of wet bacterial cells or dried bacterial cells.

The above examples of treatment may be used alone or in combinations where appropriate. According to the present invention, such treated product can be used similarly to a lactic acid bacterium.

The above-obtained bacterial cells and/or a treated product of a lactic acid bacterium can be used alone or in combination with other ingredients as a composition for preparing the functional peptide(s) from animal milk or milk proteins. Therefore, the present invention provides a composition for preparing the functional peptide(s) comprising at least one lactic acid bacterium or a treated product thereof. The composition of the present invention enables the production of the functional peptide(s) by the fermentation of animal milk or milk proteins. The composition of the present invention contains the lactic acid bacterium and/or the treated product as described above as an active ingredient. It may contain bacterial cells and/or a treated product of a single lactic acid bacterium. Alternatively, it may contain bacterial cells and/or a treated product obtained from two or more different lactic acid bacteria. Further, it may contain a combination of two or more treated products of lactic acid bacterium or bacteria treated in different ways. The composition of the present invention preferably contains bacterial cells of a lactic acid bacterium or bacteria in an amount of 1 x 10⁷ cells/ml or more.

Further, in addition to a lactic acid bacterium used as active ingredient(s), one or more additives and excipients known in the art can be added to the composition of the present invention if the desired activity is not inhibited. The composition of the present invention may also contain an additive (for example, glutamic acid, and a sugar such as glucose) which promotes the fermentation of a lactic acid bacterium. The form of the composition of the present invention includes, but not particularly limited to, suspensions, granules, powders, capsules, and other forms. The content of the active ingredient (lactic acid bacterium) in the composition of the present invention depends on its form. The content may be generally 0.0001% to 99% by mass, preferably 0.001% to 80% by mass, and more preferably 0.001% to 75% by mass as the amount of the lactic acid bacterium. The amount of the lactic acid bacterium contained in the composition of the present invention may be about 10⁷ cells/g to about 10¹² cells/g.

Fermented milk (fermented milk composition) which contains the functional peptide(s) with a high concentration can be obtained by fermenting animal milk or milk proteins using the lactic acid bacterium or the treated product thereof as described above. The functional peptide(s) can be prepared by fermenting animal milk or milk proteins using the lactic acid bacterium or the treated product thereof described above and isolating the functional peptide(s) from a resultant fermented milk.

The fermentation can be carried out by adding at least one lactic acid bacterium or a treated product thereof to animal milk or milk protein, and culturing the mixture under appropriate conditions. Examples of the animal milk may include mammalian milk such as cow milk, goat milk, or horse milk, or processed milk such as defatted milk, reconstituted milk, or condensed milk, and one type of milk or a combination of a plurality of milk or processed milk may be used. The solid concentration of the milk is not particularly limited. For example, the solid concentration of nonfat milk when defatted milk is used is approximately 3 to 15% by mass, preferably 6 to 15% by mass. The animal milk may be subjected to sterilization treatment before fermentation. The animal milk may contain such additives as to promote the fermentation of lactic acid bacteria (for example, glutamic acid and a saccharide such as glucose). Alternatively, milk protein can also be isolated from animal milk, followed by performing fermentation using the milk protein component as a substrate. The milk protein component includes, for example, casein protein.

The lactic acid bacterium added to the animal milk as a starter is preferably precultured lactic acid bacterium. The amount of the lactic acid bacterium added is not limited, and it may be typically 0.005 to 10% by mass, preferably 0.05 to 5% by mass, in terms of dried lactic acid bacterial cells. The lactic acid bacterium used may be a lactic acid bacterium culture as it is, or a treated product thereof. Specifically, it may be cultured lactic acid bacterium separated from the medium by filtration or centrifugation or may be one stored by freezing or lyophilization after separation from the medium.

The conditions of fermentation are almost the same as the culture conditions for lactic acid bacteria, and the fermentation is carried out under anaerobic conditions at 20°C to 50°C, preferably at 25°C to 42°C, more preferably 28°C to 37°C. The temperature conditions can be adjusted using a thermostatic bath, a mantle heater, a jacket, or the like. The fermentation treatment can be performed in the format of static culture, shake culture, tank culture, or the like. The fermentation period may be 3 to 96 hours, preferably 12 to 36 hours. In Examples described hereinbelow, it was determined that functional peptides were abundantly produced 8 to 48 hours, particularly 10 to 16 hours after the start of fermentation at 32°C. It was also determined that the functional peptides are abundantly produced 6 to 48 hours, particularly 7 to 16 hours after the start of fermentation at 37°C. Thus, the fermentation period may be preferably 8 to 48 hours, particularly 10 to 16 hours at 32°C, and 6 to 48 hours, particularly 7 to 16 hours at 37°C. The fermentation is preferably carried out while measuring the pH and acidity in the fermenter, and the pH is preferably maintained at 4.0 to 8.0.

Fermented milk can be obtained in the way as described above. For the purpose of the present invention, "fermented milk (composition)" means both of the whole fermented product and the fermented supernatant, and may contain lactic acid bacterial cells used for the fermentation or a disrupted product of the bacterial cells, animal milk or milk protein as a substrate, and other components. Whether the functional peptide(s) is/are contained in the resultant fermented milk or not can be determined by a technique well-known in the art, for example, high-performance liquid chromatography, mass spectrometry, or the like or a combination of these techniques.

The functional peptide(s) is/are contained in the resultant fermented milk composition, and the concentration may be 0.1 µg/ml or more, preferably 0.5 µg/ml or more, more preferably 1 µg/ml or more, still more preferably 4 µg/ml or more, particularly 5 µg/ml to 50 µg/ml, or more. When the fermented milk composition contains a plurality of the functional peptides, the concentrations of the functional peptides may be different from each other. For example, the fermented milk composition of the present invention may contain 4 µg/ml or more of the peptide consisting of SEQ ID NO: 1 and 1 µg/ml or more of the peptide consisting of SEQ ID NO: 3.

The fermented milk composition obtained as described above can be directly used, or it may be further subjected to a treatment as long as it contains the functional peptide(s) of interest. The treated product of the fermented milk may be further subjected to treatment. Examples of such treatment are described below.

Fermented milk and/or a treated product thereof can be prepared in the form of suspension or diluted solution by suspension or dilution in an adequate solvent. Examples of a solvent that can be used include water, physiological saline, and phosphate buffer saline (PBS).

A sterilized product can be prepared by sterilization treatment of fermented milk and/or a treated product thereof. In order to subject fermented milk and/or a treated product thereof to sterilization treatment, for example, a known technique such as filtration sterilization, radiation disinfection, superheat disinfection, or pressure disinfection can be used.

A heated product can be prepared by heat treatment of fermented milk and/or a treated product thereof. In order to prepare such heated product, high temperature treatment (for example, at 80 °C to 150 °C) of fermented milk and/or a treated product thereof is performed for a certain period of approximately 10 minutes to 1 hour (e.g., approximately 10 to 20 minutes).

A supernatant (whey) of fermented milk and/or a treated product thereof can be prepared by filtering or centrifuging the fermented milk and/or treated product thereof.

Fermented milk and/or a treated product thereof can be processed into the form of a powdery product (powder) or granular product via drying. Drying methods include, but not particularly limited to, spray drying, drum drying, vacuum drying, and lyophilization, which can be used alone or in combination. Upon drying, excipients may be added according to need conventionally.

The above examples of treatment may be used alone or in combinations where appropriate. According to the present invention, such treated product of fermented milk is encompassed in "fermented milk (composition)."

The fermented milk composition obtained as described above contains at least one functional peptide. It may contain several types of functional peptides in combination.

Further, a functional peptide(s) or a fraction containing it(them) may be purified from the obtained fermented milk or a treated product thereof by a known separation/purification method. The functional peptide(s) can be isolated and purified by a conventional biochemistry method which has been used in the isolation and purification of a protein or peptide, for example, precipitation using ammonium sulfate, gel chromatography, ion-exchange chromatography, affinity chromatography and others, which may be used alone or in combination. The thus isolated functional peptide(s) can be prepared as a composition by a method known in the art, for example, by a formulation method described below.

The fermented milk composition obtained as described above or the functional peptide(s) or a composition thereof can be applied in uses for which the properties of the functional peptide(s) are useful. For example, the peptide consisting of the amino acid sequence shown in SEQ ID NO: 5 or SEQ ID NO: 6 has a brain function-improving action. So, the fermented milk composition or the functional peptide(s) or a composition thereof can be used to improve brain function, thereby preventing amnesia as well as enhancing memory. The fermented milk composition or the functional peptide(s) or a composition thereof can also be used for treating or preventing symptoms and diseases caused by decreased brain function, for example, diseases or symptoms such as depression, schizophrenia, deliria, and dementias (cerebrovascular dementia, Alzheimer disease, and the like).

Further, additives described below and other known brain function improving agents can be added alone or in combination thereof to the fermented milk composition prepared as described above, or the functional peptide(s) or a composition thereof if the desired activity is not inhibited. The fermented milk composition or the functional peptide(s) or a composition thereof can be formulated by a method known in the art.

The form of the composition includes, but not particularly limited to, a form of a fermented milk, that is, a suspension which is directly obtained after the fermentation, or a form such as tablets, capsules, granules, powders, dust formulations, syrups and dry syrups, which can be obtained by the treatment using a technique known in the art. The composition may be preferably in the form of an oral formulation. In addition, a liquid formulation such as a suspension may be in a form which may be dissolved or suspended in water or a different adequate medium to prepare the liquid formulation immediately before use. When the composition is formulated into tablets or granules, coating may be performed by a known method.

The composition in the above form can be prepared according to a conventional method by formulating conventionally-used additives such as excipients, disintegrators, binders, wetting agents, stabilizers, buffering agents, lubricants, preservatives, surfactants, sweeteners, flavoring agents, aromatics, acidulants, and coloring agents into the fermented milk obtained by fermentation or the functional peptide(s). For example, in a case in which the fermented milk or the functional peptide(s) is prepared as a pharmaceutical composition or for health promotion, a pharmaceutically acceptable carrier or an additive can be incorporated into the composition. Examples of such pharmaceutically acceptable carriers and additives include water, pharmaceutically acceptable organic solvents, collagen, polyvinyl alcohol, polyvinyl pyrrolidone, carboxyvinyl polymers, sodium alginate, water-soluble dextran, water-soluble dextrin, carboxymethyl starch sodium, pectin, xanthan gum, arabic gum, casein, gelatin, agar, glycerin, propylene glycol, polyethylene glycol, vaseline, paraffin, stearyl alcohol, stearic acid, human serum albumin, mannitol, sorbitol, lactose, and surfactants acceptable as pharmaceutical additives.

Further, the composition may further contain a variety of additives used for production of medicines, food or drink products, or feeds and other various substances. Examples of such substances and additives include a variety of fats and oils (e.g., plant oils such as soybean oil, corn oil, safflower oil, and olive oil, and animal fat and oil such as beef fat or sardine oil), herbal medicines (e.g., royal jelly and ginseng), amino acids (e.g., glutamine, cysteine, leucine, and arginine), polyalcohols (e.g., ethylene glycol, polyethylene glycol, propylene glycol, glycerin, and sugar alcohols such as sorbitol, erythritol, xylitol, maltitol, and mannitol), natural polymers (e.g., arabic gum, agar, water-soluble corn fibers, gelatin, xanthan gum, casein, gluten or gluten hydrolysate, lecithin, starch, and dextrin), vitamins (e.g., vitamin C and vitamin Bs), minerals (e.g., calcium, magnesium, zinc, and iron), dietary fibers (e.g., mannan, pectin, and hemicellulose), surfactants (e.g., glycerin esters of fatty acid and sorbitan esters of fatty acid), purified water, excipients (e.g., glucose, cornstarch, lactose, and dextrin), stabilizing agents, pH adjusting agents, antioxidants, sweeteners, flavoring agents, acidulants, coloring agents, and aromatics.

Further, in addition to the above active ingredients, a functional ingredient, a pharmaceutical ingredient or an additive can be incorporated into the composition:
Food ingredients:
Ginkgo leaf extract, arachidonic acid (ARA), GABA, theanine, ceramide, caffeine, carnitine, α-glycerylphosphorylcholine (α-GPC), *Bacopa monniera,* DHA-bound phospholipids, phosphatidylserine (PS), phosphatidylcholine, St. John's wort, astaxanthin, niacin, pyrroloquinoline quinone (PQQ), and coenzyme Q10 (CoQ10); Unsaturated fatty acids such as, for example, docosahexaenoic acid (DHA) and eicosapentaenoic acid (EPA); Polyphenols such as, for example, resveratrol, chlorogenic acid, and catechins.
Pharmaceuticals:
Therapeutic drugs for dementia such as, for example, an acetylcholinesterase inhibitor (donepezil, galanthamine, rivastigmine, tacrine, or the like) and an NMDA receptor antagonist (memantine or the like);
Antianxiety drugs such as, for example, a benzodiazepine antianxiety drug;
Antidepressant drugs such as, for example, a selective serotonin reuptake inhibitor (SSRI), serotonin/norepinephrine (noradrenaline) reuptake inhibitor (SNRI), a tricyclic antidepressant (TCA), a tetracyclic antidepressant, a triazolopyridine antidepressant (SARI), a monoamine oxidase inhibitor (MAO inhibitor), a noradrenergic/specific serotonergic antidepressant (NaSSA), and a norepinephrine/dopamine reuptake inhibitor (NDRI);
Antipsychotic drugs; and
Hypnotics.

The amount of these functional ingredients, pharmaceutical ingredients or additives may be determined appropriately depending on their types and desired dosages.

Subjects of administration or intake of the composition prepared as described above may be vertebrate animals. Specific examples thereof include mammals such as humans, primates (e.g., monkeys and chimpanzees), livestock animals (e.g., cattle, horses, pigs, sheep, and fowl), pet animals (e.g., dogs and cats), and experimental animals (e.g., mice and rats). Further, such subjects can be reptiles and birds. Particularly preferable subjects are subjects for whom the intake of the functional peptide(s) is desired, for example, humans who need the treatment for a condition and disease caused by decreased brain function. The dose of administration or intake of the composition may depends on the age and body weight of a subject, an administration/intake route, the number of doses for administration/intake, the purpose of administration and other factors, and can be adjusted extensively at the discretion of those skilled in the art to achieve a desired effect. The content of the functional peptide(s) contained in the composition is not particularly limited and can be adequately adjusted in accordance with the degree of ease of production, and the preferable daily dose, for example. The daily dose may be administered/taken in a single dose, or it may be divided into several doses. In addition, the frequency of administration or intake is not particularly limited, and it can be adequately selected depending on various conditions such as an administration/intake route, the age and body weight of a subject, and desired effects. The administration/intake route of the composition is not particularly limited, and is preferably oral administration/intake. For example, the composition may be orally administered or taken by incorporating it into a food and drink or feed, or formulating into a tablet or granule.

The composition prepared as described above may be used in combination with an additional medicine or an additional treatment or prevention method. Such additional medicine and the composition may be formulated into a single formulation. Alternatively, they may be formulated into separate formulations so as to be administered simultaneously or at intervals.

In addition, the fermented milk composition prepared according to the present invention or the functional peptide or a composition containing the functional peptide is safe and thus is easily used for long-term continuous intake. Therefore, it can also be added in food or drink products or feeds. The fermented milk composition or the functional peptide or composition can be continuously taken by adding it to a variety of food or drink product with the expectation of a variety of effects.

According to the present invention, a functional food or drink can be prepared by blending the fermented milk composition prepared as described above, or the functional peptide(s) or a composition containing it into a food or drink. The food or drink product of the present invention also includes beverages and supplements. Examples of the food or drink product of the present invention include all food or drink products for health promotion by the functional peptide(s), for example, food or drink products such as health food or drink products, functional food or drink products, food for specified health use, and supplements (e.g., nutritional food, health supplements and nutritional supplements).

Specific examples of food or drink products include health food or drink products and nutritional supplements in preparation forms such as liquid diets (e.g., tube enteral nutritional supplements), tablet candies, tablets, chewable tablets, dust formulations, powders, capsules, granules, and tonic drinks; tea beverages such as green tea, oolong tea, and black tea; drinks or beverages such as soft drinks, jelly beverages, isotonic beverages, milk beverages, carbonated beverages, vegetable beverages, juice beverages, fermented vegetable beverages, fermented juice beverages, fermented milk (e.g., drink yogurt, set yogurt), fermented milk beverages (sterile), lactic acid bacteria beverages, concentrate beverage, concentrate solid portion, milk beverages (e.g., coffee milk), beverages containing drink powders, cocoa beverages, milk, and purified water; spreads such as butter, jam, dried seasoning products, and margarine; mayonnaise; shortening; custard; dressings; bread; boiled rice; noodles; pasta; miso soup; tofu; yogurt; soup or sauce; and sweets (e.g., biscuits and cookies, chocolate, candies, cake, ice cream, chewing gum, and tablets).

According to the present invention, the food or drink product can be produced according to a conventional method by adding other food materials used for production of the above food or drink products, various nutrients, various vitamins, minerals, dietary fibers, and various additives (e.g., taste components, sweeteners, acidulants such as organic acids, stabilizers, and flavors), in addition to the fermented milk composition or the functional peptide(s). Those skilled in the art can adequately determine the amount of the fermented milk or functional peptide(s) contained in the food or drink in consideration of the form of the food or drink product and the taste or texture that are required.

The food or drink product may be produced by an appropriate method available by those skilled in the art. For example, the fermented milk or functional peptide(s) can be prepared in a liquid, gel, solid, powder, or granule form and then incorporated into a food or drink product. Alternatively, the fermented milk or functional peptide(s) may be mixed or dissolved directly into raw materials for a food or drink product. The fermented milk or functional peptide(s) may be applied to, coated onto, infiltrated into, or sprayed onto a food or drink product. The fermented milk or functional peptide(s) may be dispersed uniformly or distributed unevenly in a food or drink product. A capsule containing the fermented milk or functional peptide(s) may be prepared. An edible film or food coating agent may be wrapped around the fermented milk or functional peptide(s). Alternatively, the fermented milk or functional peptide(s) may be prepared into a form such as a tablet after the addition of an appropriate excipient and others. The food or drink product may further be processed. A method for preparing such treated product is also encompassed within the scope of the present invention.

In the method of preparing the food or drink product according to the present invention, a variety of additives as routinely used in food or drink products may be employed. Examples of the additives include, but not limited to, color formers (e.g., sodium nitrite), coloring agents (e.g., gardenia pigments and Red 102), flavors (e.g., orange flavors), sweeteners (e.g., stevia and aspartame), preservatives (e.g., sodium acetate and sorbic acid), emulsifiers (e.g., sodium chondroitin sulfate and propylene glycol esters of fatty acid), antioxidants (e.g., disodium EDTA and vitamin C), pH adjusters (e.g., citric acid), chemical seasonings (e.g., sodium inosinate), thickeners (e.g., xanthan gum), swelling agents (e.g., calcium carbonate), antifoaming agents (e.g., calcium phosphate), binding agents (e.g., sodium polyphosphate), nutrition-enriching agents (e.g., calcium-enriching agents and vitamin A), and excipients (e.g., water-soluble dextrin). Functional raw materials such as *Panax ginseng* extracts, *Acanthopanax senticosus Harms* extracts, eucalyptus extracts, or du zhong tea extracts may further be added.

The food or drink product prepared as described above has a variety of functions based on the functional peptide(s). In addition, it is safe, and thus there is no concern about side effects.

Further, the fermented milk composition or the functional peptide or a composition thereof can be formulated not only into food or drink products for humans but also into feeds for animals such as livestock (e.g., cattle and pigs), racehorses, and pets (e.g., dogs and cats). Feeds are substantially equivalent to food or drink products except that they are given to non-human subjects. Therefore, the above descriptions of food or drink products can be applied mutatis mutandis to feeds.

In addition, based on a peptide prepared by the method of the present invention, a peptide comprising an amino acid sequence shown in SEQ ID NO: 5 or SEQ ID NO: 6 can also be generated by deletion, substitution or addition of one or several amino acids in the prepared peptide using a method known in the art.

### EXAMPLES

The present invention will be described below in further detail with reference to the examples and drawings. However, the scope of the invention is not limited to these examples.

### [EXAMPLE 1]

In this Example, fermented milk was prepared using lactic acid bacteria. 9.0% (w/w) reconstituted skim milk autoclaved at 105°C for 10 minutes for sterilization was used as a milk medium. Then, the prepared milk medium was inoculated with fermented milk containing lactic acid bacteria, at a concentration of 3%, which was then cultured at 37°C for 24 hours; the resultant fermented milk was used as a starter. In main culture, the starter was added to the milk medium at a concentration of 3%, and fermentation was carried out at 37°C for 24 hours. For a sample whose fermentation progressed slowly, the fermentation time was prolonged to 48 to 72 hours.

*Lactobacillus helveticus* CM4 strain (Accession Number: FERM BP-6060) was used as a starter, and *Lactobacillus helveticus* JCM1120 strain, *Lactobacillus helveticus* JCM1004 strain, *Lactobacillus delbrueckii bulgaricus* JCM1002 strain, *Lactobacillus acidophilus* JCM1132 strain, *Lactobacillus casei* JCM1134, and *Streptococcus thermophilus* JCM20026 strain were also evaluated for reference.

### [EXAMPLE 2]

In this Example, the content of peptides (SEQ ID NOS: 1 to 4) in the fermented milk supernatant was measured. Specifically, 600 µl of ultrapure water, 200 µl of acetonitrile, and 100 µl of a 10% aqueous trichloroacetic acid solution were added to 100 µl of the fermented milk obtained in Example 1, and then thoroughly mixed. Subsequently, the mixture was centrifuged at 15,000 rpm for 10 minutes and the supernatant was recovered, and the resultant solution was diluted 50 times its initial concentration with a 20% aqueous acetonitrile solution, and quantitatively analyzed for each peptide using a high-performance liquid chromatograph triple quadrupole mass spectrometer (LC/MS/MS, Waters TQD). Each component was separated by gradient analysis using a reverse phase ODS column as a separation column and a 0.1% aqueous formic acid solution and 0.1% formic acid-containing acetonitrile as eluents, and the quantitation was carried out by generating a calibration curve using a synthetic peptide as a standard and calculating the content of the peptides.

The quantitation results of the peptides (SEQ ID NOS: 1 to 4) in the fermented milk supernatants are shown in Table 1.

**[Table 1]**

| Strain Name | Temperature [°C] | Period [hr] | | SEQ ID NO:1 | SEQ ID NO:2 | SEQ ID NO:3 | SEQ ID NO:4 |
|---|---|---|---|---|---|---|---|
| *Lactobacillus helveticus* CM4 | 37 | 24 | | 16.4 | 22.3 | 12.2 | 9.1 |
| *Lactobacillus helveticus* JCM1120 | 37 | 24 | | 10.0 | 49.0 | 5.9 | 2.4 |
| *Lactobacillus helveticus* JCM1004 | 37 | 24 | | 5.6 | 11.8 | 1.7 | 0.6 |
| *Lactobacillus delbrueckii bulgaricus* JCM1002 | 37 | 24 | | 12.9 | 18.5 | 0.0 | 0.0 |
| *Lactobacillus casei* JCM1134 | 37 | 72 | | 0.6 | 2.6 | 0.0 | 0.0 |
| *Lactobacillus acidophilus* JCM1132 | 37 | 48 | | 0.4 | 2.1 | 3.7 | 0.1 |
| *Streptococcus thermophilus* JCM20026 | 37 | 24 | | 0.0 | 0.0 | 0.1 | 0.1 |

| | | | | | | | |
|---|---|---|---|---|---|---|---|
| Concentration in fermented milk [µg/ml] | | | | | | | |

The results of Table 1 showed that the peptides (SEQ ID NOS: 1 to 4) were produced by the fermentation of lactic acid bacteria. Particularly, the use of each of *Lactobacillus helveticus* and *Lactobacillus delbrueckii bulgaricus* was demonstrated to result in the high production of these peptides. In addition, the concentration of each peptide in the fermented milk was shown to be 0.1 µg/ml to about 50 µg/ml.

### [EXAMPLE 3]

In this Example, changes in the amounts of peptides (SEQ ID NOS: 1 to 4) over time during fermentation in the fermented milk supernatant were examined. Specifically, 9.0% (w/w) reconstituted skim milk autoclaved at 105°C for 10 minutes for sterilization was used as a milk medium. Then, the prepared milk medium was inoculated with fermented milk containing *Lactobacillus helveticus* CM4 strain (Accession Number: FERM BP-6060), at a concentration of 3%, and then cultured at 37°C for 24 hours; the resultant fermented milk was used as a starter. In main culture, the starter was added to the milk medium at a concentration of 3%, and then fermented at 32°C or 37°C. The fermented media were collected at 1, 2, 4, 8, 12, 16, 24, and 48 hours of fermentation. Thereafter, the same pretreatment as in Example 2 was carried out, and the peptides (SEQ ID NOS: 1, 3, and 4) were quantitatively analyzed using a high-performance liquid chromatograph triple quadrupole mass spectrometer (LC/MS/MS, Waters TQD). As in Example 2, each component was separated by gradient analysis using a reverse phase ODS column as a separation column and a 0.1% aqueous formic acid solution and 0.1% formic acid-containing acetonitrile as eluents, and the quantitation was carried out by generating a calibration curve using a synthetic peptide as a standard and calculating the content of the peptides.

For comparison, tripeptides (Val-Pro-Pro and Ile-Pro-Pro) were also similarly quantitatively analyzed which were known as biologically active peptides produced in *Lactobacillus helveticus-fermented* milk and had a blood-pressure-lowering action.

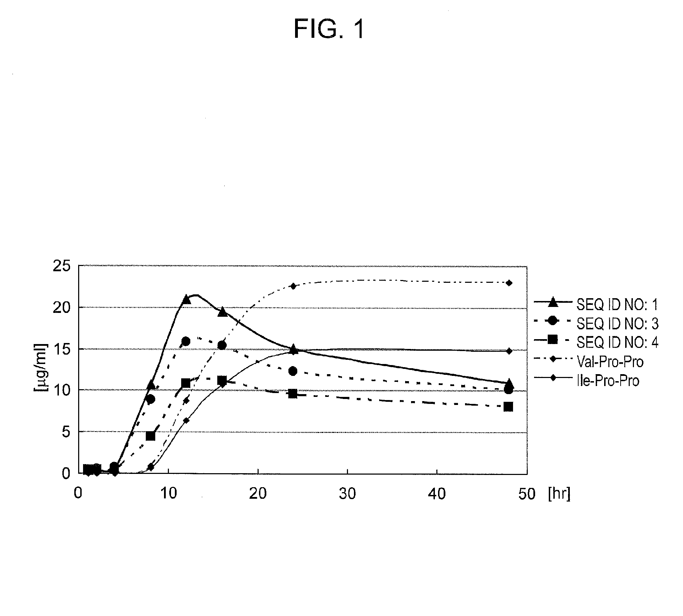

The quantitation results of the peptides in each supernatant are shown in Table 2 and FIG. 1.

**[Table 2]**

| Fermentation Period [h] | | 1 | 2 | 4 | 8 | 12 | 16 | 24 | 48 |
|---|---|---|---|---|---|---|---|---|---|
| SEQ ID NO:1 | 32°C | 0.5 | 0.5 | 0.6 | 10.7 | 21.0 | 19.6 | 15.0 | 11.0 |
| | 37°C | 0.5 | 0.5 | 1.4 | 15.2 | 13.2 | 12.4 | 9.9 | 9.4 |
| SEQ ID NO:3 | 32°C | 0.5 | 0.6 | 0.7 | 8.9 | 15.9 | 15.4 | 12.3 | 10.1 |
| | 37°C | 0.5 | 0.5 | 2.2 | 11.1 | 12.2 | 12.0 | 10.7 | 10.9 |
| SEQ ID NO:4 | 32°C | 0.4 | 0.5 | 0.5 | 4.4 | 10.9 | 11.1 | 9.6 | 8.2 |
| | 37°C | 0.5 | 0.5 | 1.1 | 6.9 | 9.0 | 8.8 | 7.9 | 8.1 |
| Val-Pro-Pro | 32°C | 0.0 | 0.0 | 0.0 | 0.9 | 8.8 | 15.6 | 22.6 | 23.1 |
| | 37°C | 0.0 | 0.0 | 0.0 | 7.7 | 15.8 | 16.6 | 17.8 | 17.8 |
| Ile-Pro-Pro | 32°C | 0.0 | 0.0 | 0.0 | 0.7 | 6.4 | 10.7 | 14.7 | 14.8 |
| | 37°C | 0.0 | 0.0 | 0.1 | 4.8 | 9.4 | 9.7 | 10.4 | **10.5** |

| | | | | | | | | | |
|---|---|---|---|---|---|---|---|---|---|
| Concentration in fermented milk [µg/m] | | | | | | | | | |

The above results showed that these peptides were produced 3 to 96 hours after the start of culture. Specifically, the functional peptides were abundantly produced 8 to 48 hours, particularly 10 to 16 hours after the start of fermentation at 32°C, and the functional peptides were abundantly produced 6 to 48 hours, particularly 7 to 16 hours after the start of fermentation at 37°C. Thus, they were found to be highly produced by a shorter time (approximately 8 to 12 hours) of fermentation than the time for the previously known biologically active peptides. In addition, the concentration of the peptides in the fermented milk was 0.5 µg/ml or more at 2 hours after the start of culture and 1 µg/ml or more, or even 5 µg/ml or more, at 8 hours (32°C) or 6 hours (37°C) after the start of culture.

### [EXAMPLE 4]

In this Example, an amnesia-preventing action of Asn-Ile-Pro-Pro-Leu-Thr-Gln-Thr-Pro-Val-Val-Val-Pro-Pro-Phe-Leu-Gln-Pro-Glu (NIPPLTQTPVVVPPFLQPE; SEQ ID NO: 1) is to be demonstrated.

Male ddY mice (about 7 weeks old) were used (n = 15 to 75), and provided with food and water ad libitum. Asn-Ile-Pro-Pro-Leu-Thr-Gln-Thr-Pro-Val-Val-Val-Pro-Pro-Phe-Leu-Gln-Pro-Glu (SEQ ID NO: 1) was used as a test substance in amounts of 0.05 nmol/kg body weight (0.1 µg/kg body weight), 0.5 nmol/kg body weight (1 µg/kg body weight), 1.5 nmol/kg body weight (3 µg/kg body weight), 5 nmol/kg body weight (10 µg/kg body weight), 50 nmol/kg body weight (100 µg/kg body weight), and 500 nmol/kg body weight (1,000 µg/kg body weight). The test substance was administered at a single dose orally to mice 60 minutes before performing a Y-shaped maze test for evaluating spontaneous alternation behavior. Thirty minutes before performing the Y-shaped maze test, scopolamine was subcutaneously administered in an amount of 1 mg/kg body weight into the back to induce brain dysfunction (memory disorder and/or cognition disorder) in mice. In the Y-shaped maze test, as an experiment device, a Y-shaped maze was used in which the length for each arm was 40 cm; the wall height was 12 cm; the floor width was 3 cm; the upper part width was 10 cm; and three arms were connected to each other at an angle of 120°. Each mouse was placed in the end of any of the arms of the Y-shaped maze and allowed to explore freely in the maze over 8 minutes, and the sequence of the arms to which the mouse moved was recorded. The number of movements of the mouse to the arms within the measurement time was counted and used as the total number of entries; in the sequence, the combination in which three different arms were selected in succession (for example, with the three arms respectively called A, B, and C, if the sequence of the arms entered is ABCBACACB, the count is 4 inclusive of overlapping) was investigated, and the count number was used as the number of spontaneous alternation behaviors. The change in spontaneous alternation behavior (%) was calculated by dividing the number of spontaneous alternation behaviors by a number obtained by subtracting 2 from the total number of entries, and multiplying the resultant number by 100, and the percentage was used as an indicator of the spontaneous alternation behavior. A higher value of the indicator suggests better maintenance of short-term memory. The measured values were expressed as mean ± standard error for each group. The significance of difference between the control group and the scopolamine control group was tested by Student's t-test. The significance of difference between the scopolamine control group and the peptide-administered group was tested by Dunnett's multiple comparison test after one-way analysis of variance.

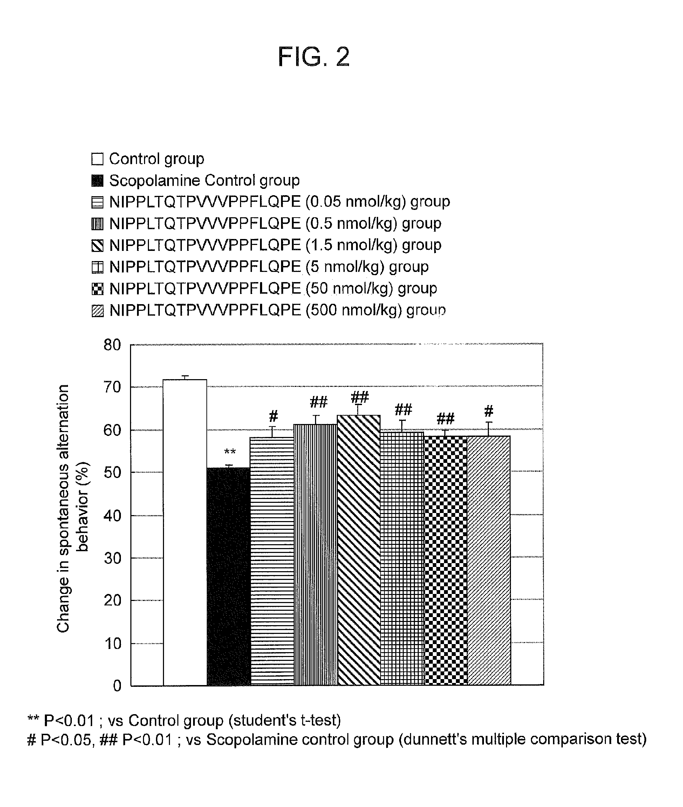

The results are shown in FIG. 2. NIPPLTQTPVVVPPFLQPE (SEQ ID NO: 1) was shown to have an amnesia-preventing action in the range of 0.05 nmol/kg body weight to 500 mnol/kg body weight (0.1 µg/kg body weight to 1,000 µg/kg body weight).

### [EXAMPLE 5]

In this Example, amnesia-preventing actions of Asn-Ile-Pro-Pro-Leu-Thr-Gln-Thr-Pro-Val-Val-Val-Pro-Pro-Phe-Leu-Gln-Pro-Glu (NIPPLTQTPVVVPPFLQPE)-related peptides are to be demonstrated.

Male ddY mice (about 7 weeks old) were used (n = 15 to 45), and provided with food and water ad libitum. As test substances, Asn-Ile-Pro-Pro-Leu-Thr-Gln-Thr-Pro-Val-Val-Val-Pro-Pro-Phe-Leu-Gln-Pro-Glu (SEQ ID NO: 1) at 50 nmol/kg body weight (100 µg/kg body weight), or Asn-Ile-Pro-Pro-Leu-Thr-Gln-Thr-Pro-Val-Val-Val-Pro-Pro-Phe-Leu-Gln-Pro-Glu-Val-Met (NIPPLTQTPVVVPPFLQPEVM; SEQ ID NO: 2) at 50 nmol/kg body weight (120 µg/kg body weight), or Ile-Pro-Pro-Leu-Thr-Gln-Thr-Pro-Val-Val-Val-Pro-Pro-Phe-Leu-Gln-Pro-Glu (IPPLTQTPVVVPPFLQPE; SEQ ID NO: 7) at 50 nmol/kg body weight (100 µg/kg body weight), or Asn-Ile-Pro-Pro-Leu-Thr-Gln-Thr-Pro-Val-Val-Val-Pro-Pro-Phe-Leu-Gln-Pro (NIPPLTQTPVVVPPFLQP; SEQ ID NO: 8) at 50 nmol/kg body weight (100 µg/kg body weight), or Thr-Gln-Thr-Pro-Val-Val-Val-Pro-Pro-Phe (TQTPVVVPPF; SEQ ID NO: 9) at 50 nmol/kg body weight (50 µg/kg body weight) were used. The percentage of change in the spontaneous alternation behavior was determined as described in Example 4, and used as an indicator of the spontaneous alternation behavior. The measured values were expressed as mean ± standard error for each group. The significance of difference between the control group and the scopolamine control group was tested by Student's t-test. The significance of difference between the scopolamine control group and each peptide-administered group was tested by Dunnett's multiple comparison test after one-way analysis of variance.

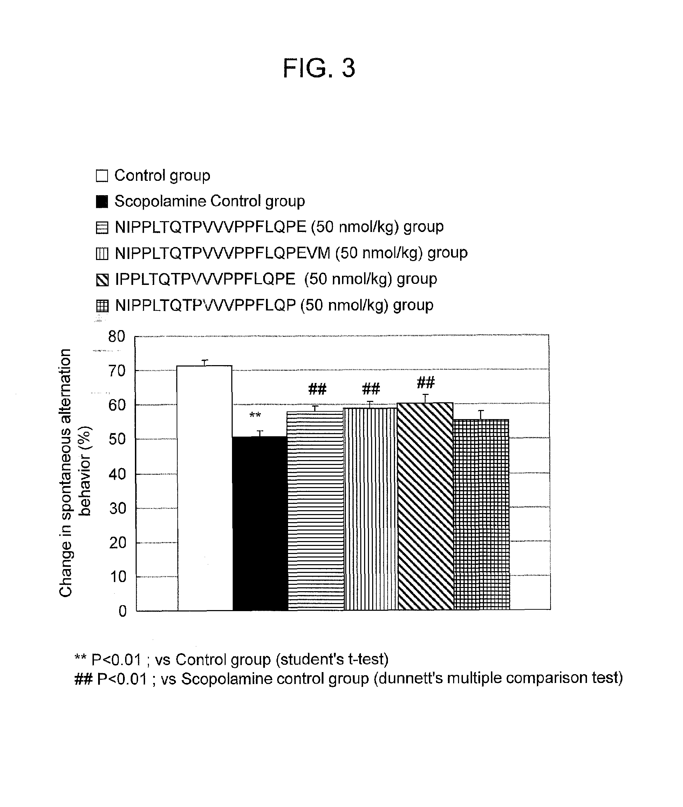

The results are shown in FIG. 3. NIPPLTQTPVVVPPFLQPE (SEQ ID NO: 1) at 50 nmol/kg body weight (100 µg/kg body weight), NIPPLTQTPVVVPPFLQPEVM (SEQ ID NO: 2) at 50 nmol/kg body weight (120 µg/kg body weight), and IPPLTQTPVVVPPFLQPE (SEQ ID NO: 7) at 50 nmol/kg body weight (100 µg/kg body weight) were shown to have amnesia-preventing actions. However, the significant difference compared to the scopolamine control group could not be confirmed for NIPPLTQTPVVVPPFLQP (SEQ ID NO: 8) at 50 nmol/kg body weight (100 µg/kg body weight) and TQTPVVVPPF (SEQ ID NO: 9) at 50 nmol/kg body weight (50 µg/kg body weight) (data not shown for SEQ ID NO: 9).

### [EXAMPLE 6]

In this Example, amnesia-preventing actions of Asn-Ile-Pro-Pro-Leu-Thr-Gln-Thr-Pro-Val-Val-Val-Pro-Pro-Phe-Leu-Gln-Pro-Glu (NIPPLTQTPVVVPPFLQPE)-related peptides are to be demonstrated.

Male ddY mice (about 7 weeks old) were used (n = 14 to 15), and provided with food and water ad libitum. As test substances, Asn-Ile-Pro-Pro-Leu-Thr-Gln-Thr-Pro-Val-Val-Val-Pro-Pro-Phe-Leu-Gln-Pro-Glu (SEQ ID NO: 1) at 500 nmol/kg body weight (1000 µg/kg body weight), or Pro-Pro-Leu-Thr-Gln-Thr-Pro-Val-Val-Val-Pro-Pro-Phe-Leu-Gln-Pro-Glu (PPLTQTPVVVPPFLQPE; SEQ ID NO: 10) at 500 nmol/kg body weight (1000 µg/kg body weight) were used. The percentage of change in the spontaneous alternation behavior was determined as described in Example 4, and used as an indicator of the spontaneous alternation behavior. The measured values were expressed as mean ± standard error for each group. The significance of difference between the control group and the scopolamine control group was tested by Student's t-test. The significance of difference between the scopolamine control group and each peptide-administered group was tested by Dunnett's multiple comparison test after one-way analysis of variance.

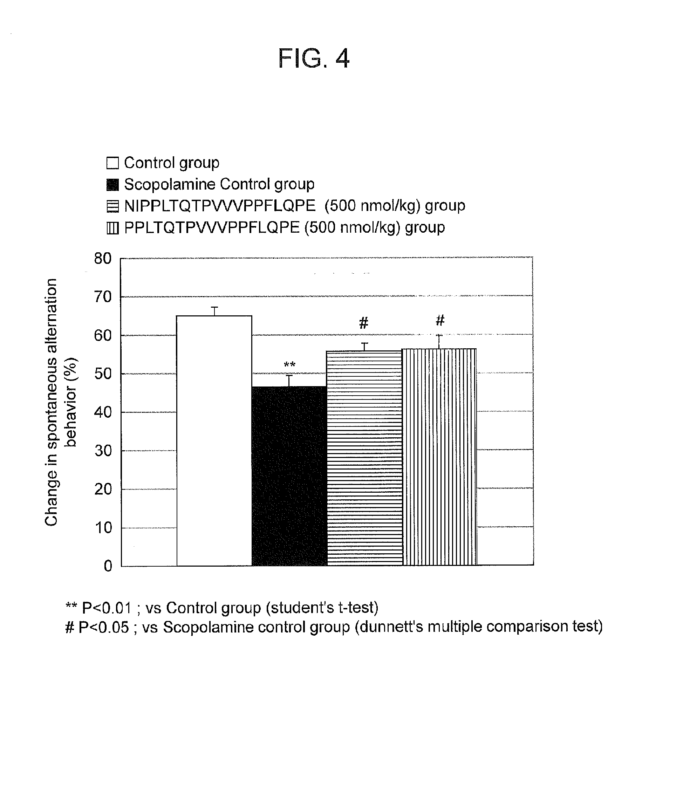

The results are shown in FIG. 4. NIPPLTQTPVVVPPFLQPE (SEQ ID NO: 1) at 500 nmol/kg body weight (1000 µg/kg body weight), and PPLTQTPVVVPPFLQPE (SEQ ID NO: 10) at 500 nmol/kg body weight (1000 µg/kg body weight) were shown to have amnesia-preventing actions.

### [EXAMPLE 7]

In this example, a memory-enhancing action of Asn-Ile-Pro-Pro-Leu-Thr-Gln-Thr-Pro-Val-Val-Val-Pro-Pro-Phe-Leu-Gln-Pro-Glu (NIPPLTQTPVVVPPFLQPE; SEQ ID NO: 1) is to be demonstrated.

Male ddY mice (about 7 weeks old) were used (n = 14 to 15), and provided with food and water ad libitum. As a test substance, 500 nmol/kg body weight (1,000 µg/kg body weight) of Asn-Ile-Pro-Pro-Leu-Thr-Gln-Thr-Pro-Val-Val-Val-Pro-Pro-Phe-Leu-Gln-Pro-Glu (SEQ ID NO: 1) was used. The test substance was administered at a single dose orally to mice 60 minutes before performing a novel object recognition test for evaluating memory retention. In the novel object recognition test, a 30 x 30 x 30 cm box was used as an experiment device. As a conditioning operation, a mouse was placed in an experiment device in which a floorcloth was laid for 5 minutes, and allowed to explore freely in the device. A training trial was performed the day following the conditioning operation. In the training trial, 2 out of 3 objects were selected and placed in the experiment device (the objects were placed at positions 8 cm from the walls on the both sides along the central line of the floor, and the positions were called X1 and X2.). For the selection of the objects to be placed, the objects were randomly selected in advance to prevent bias among the animals and among the groups. Sixty minutes after orally administering the test substance or water, a mouse was placed in the experiment device for 5 minutes, and the time (second) was measured during which the mouse explored by approaching each object to be within 1 cm therefrom. A retention trial was performed 48 hours after the training trial. In the retention trial, 2 objects were placed in the experiment device as in the training trial; however, 1 of the objects was substituted for a different object (a novel object) from that used in the training trial, and the position thereof was called Y. (For example, when an object A was placed in X1 and an object B in X2 in the training trial, an object C was placed in place of the object A in the retention trial, and the position thereof was called Y.) In the training trial and the retention trial, the time (second) was measured during which each mouse explored by approaching each object to be within 1 cm therefrom. (However, the state in which a mouse rides on an object is excluded.) The percentages of the times were determined during which two objects were explored in each of the training trial and the retention trial. The percentage (%) of the exploration time for each object was expressed as mean ± standard error for each of the groups. The significance of difference between the control group and the peptide group was tested by Student's t-test for the percentage of the exploration time for the novel object (the object placed at Y) in the retention trial and the percentage of the exploration time for the object (the object placed at X1 or X2) placed at the position at which the novel object is placed in the training trial.

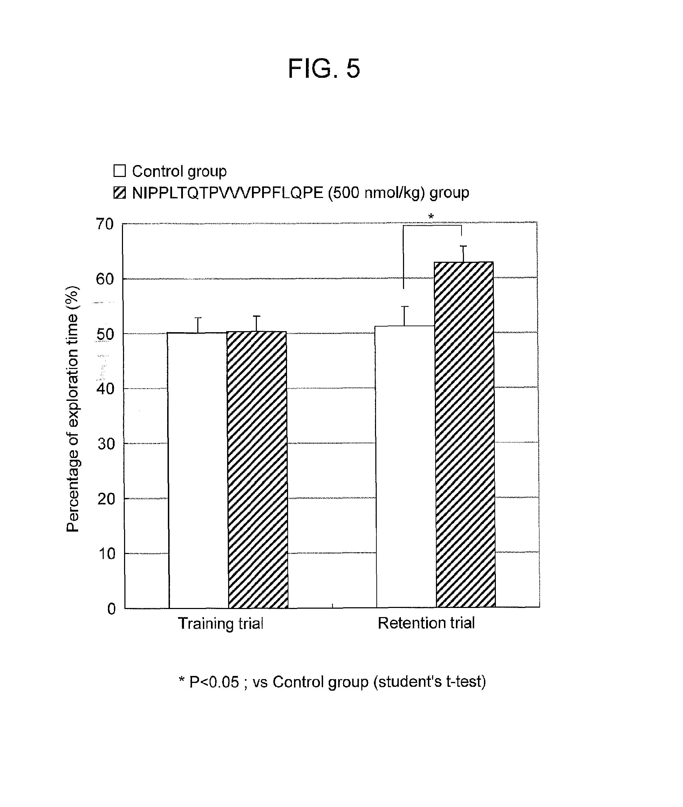

The results are shown in FIG. 5. NIPPLTQTPVVVPPFLQPE (SEQ ID NO: 1) was shown to have a memory-enhancing action at 500 nmol/kg body weight (1,000 µg/kg body weight).

### [EXAMPLE 8]

In this Example, an amnesia-preventing action of Asn-Ile-Pro-Pro-Leu-Thr-Gln-Thr-Pro-Val-Val-Val-Pro-Pro-Phe-Leu-Gln-Pro-Glu (NIPPLTQTPVVVPPFLQPE; SEQ ID NO: 1)-related peptide is to be demonstrated.

Male ddY mice (about 7 weeks old) were used (n = 27 to 40), and provided with food and water ad libitum. As a test substance, Thr-Gln-Thr-Pro-Val-Val-Val-Pro-Pro-Phe-Leu-Gln-Pro-Glu (TQTPVVVPPFLQPE; SEQ ID NO: 11) was used at 50 nmol/kg body weight (80 µg/kg body weight). The percentage of change in the spontaneous alternation behavior was determined as described in Example 4, and used as an indicator of the spontaneous alternation behavior. The measured values were expressed as mean ± standard error for each group. The significance of difference between the control group and the scopolamine control group was tested by Student's t-test. The significance of difference between the scopolamine control group and the peptide-administered group was tested by Student's t-test.

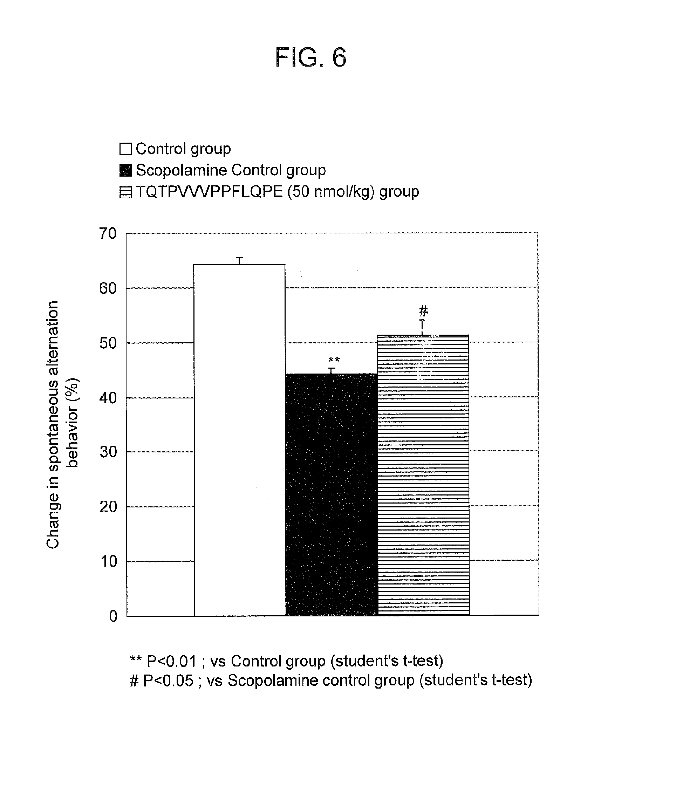

The results are shown in FIG. 6. TQTPVVVPPFLQPE (SEQ ID NO: 11) was shown to have an amnesia-preventing action at 50 nmol/kg body weight (80 µg/kg body weight).

### [EXAMPLE 9]

In this Example, amnesia-preventing actions of Asn-Ile-Pro-Pro-Leu-Thr-Gln-Thr-Pro-Val-Val-Val-Pro-Pro-Phe-Leu-Gln-Pro-Glu (NIPPLTQTPVVVPPFLQPE)-related peptides are to be demonstrated.

Male ddY mice (about 7 weeks old) were used (n = 11 to 40), and provided with food and water ad libitum. As test substances, Pro-Leu-Thr-Gln-Thr-Pro-Val-Val-Val-Pro-Pro-Phe-Leu-Gln-Pro-Glu (PLTQTPVVVPPFLQPE; SEQ ID NO: 12) at 500 nmol/kg body weight (900 µg/kg body weight), or Leu-Thr-Gln-Thr-Pro-Val-Val-Val-Pro-Pro-Phe-Leu-Gln-Pro-Glu (LTQTPVVVPPFLQPE; SEQ ID NO: 13) at 500 nmol/kg body weight (850 µg/kg body weight), or Pro-Val-Val-Val-Pro-Pro-Phe-Leu-Gln-Pro-Glu (PVVVPPFLQPE; SEQ ID NO: 14) at 500 nmol/kg body weight (630 µg/kg body weight), or Val-Val-Val-Pro-Pro-Phe-Leu-Gln-Pro-Glu (VVVPPFLQPE; SEQ ID NO: 15) at 500 nmol/kg body weight (580 µg/kg body weight) were used. The percentage of change in the spontaneous alternation behavior was determined as described in Example 4, and used as an indicator of the spontaneous alternation behavior. The measured values were expressed as mean ± standard error for each group. The significance of difference between the control group and the scopolamine control group was tested by Student's t-test. The significance of difference between the scopolamine control group and each peptide-administered group was tested by Student's t-test.

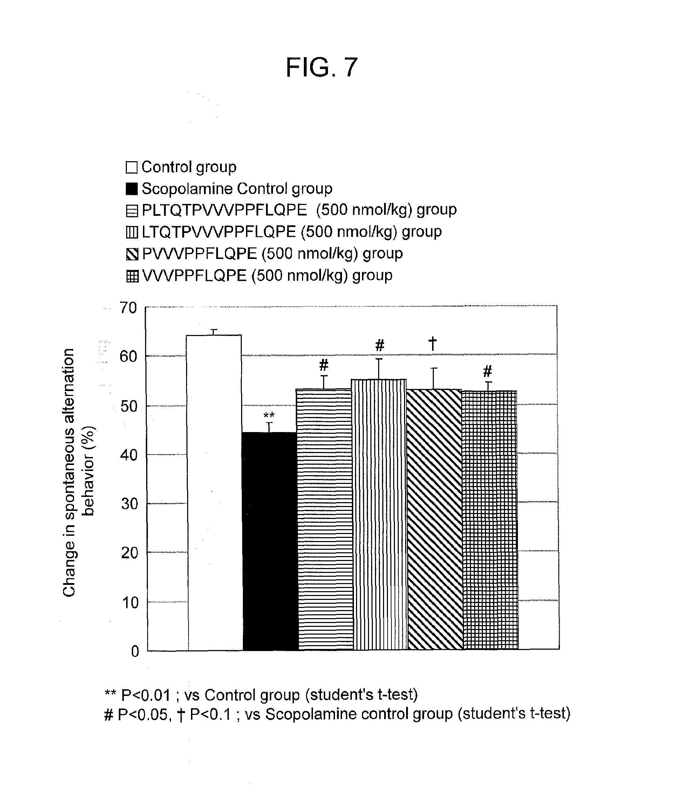

The results are shown in FIG. 7. PLTQTPVVVPPFLQPE (SEQ ID NO: 12) at 500 nmol/kg body weight (900 µg/kg body weight), LTQTPVVVPPFLQPE (SEQ ID NO: 13) at 500 nmol/kg body weight (850 µg/kg body weight), PVVVPPFLQPE (SEQ ID NO: 14) at 500 nmol/kg body weight (630 µg/kg body weight), and VVVPPFLQPE (SEQ ID NO: 15) at 500 nmol/kg body weight (580 µg/kg body weight) were shown to have amnesia-preventing actions.

### [EXAMPLE 10]

In this Example, an amnesia-preventing action of Ser-Trp-Met-His-Gln-Pro-His-Gln-Pro-Leu-Pro-Pro-Thr-Val-Met-Phe-Pro-Pro-Gln-Ser-Val-Leu (SWMHQPHQPLPPTVMFPPQS VL; SEQ ID NO: 3) is to be demonstrated.

Male ddY mice (about 7 weeks old) were used (n=15 to 30), and provided with food and water ad libitum. As a test substance, Ser-Trp-Met-His-Gln-Pro-His-Gln-Pro-Leu-Pro-Pro-Thr-Val-Met-Phe-Pro-Pro-Gln-Ser-Val-Leu (SEQ ID NO: 3) was used at 150 nmol/kg body weight (380 µg/kg body weight) and 500 nmol/kg body weight (1,280 µg/kg body weight). The percentage of change in the spontaneous alternation behavior was determined as described in Example 4, and used as an indicator of the spontaneous alternation behavior. The measured values were expressed as mean ± standard error for each group. The significance of difference between the control group and the scopolamine control group was tested by Student's t-test. The significance of difference between the scopolamine control group and the peptide-administered group was tested by Student's t-test.

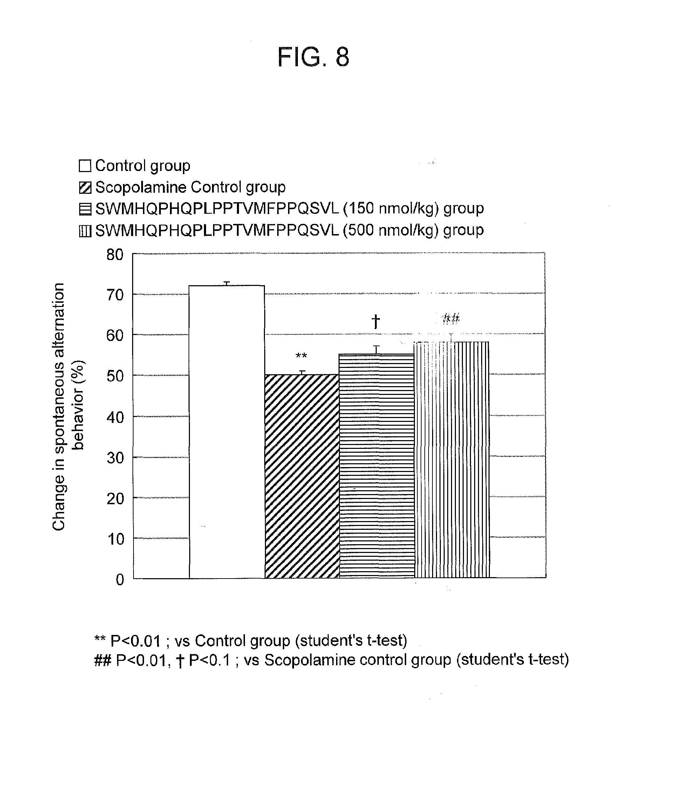

The results are shown in FIG. 8. SWMHQPHQPLPPTVMFPPQSVL (SEQ ID NO: 3) was shown to have an amnesia-preventing action at 150 nmol/kg body weight to 500 nmol/kg body weight (380 µg/kg body weight to 1,280 µg/kg body weight).

### [EXAMPLE 11]

In this Example, amnesia-preventing actions of Ser-Trp-Met-His-Gln-Pro-His-Gln-Pro-Leu-Pro-Pro-Thr-Val-Met-Phe-Pro-Pro-Gln-Ser-Val-Leu (SEQ ID NO: 3)-related peptides are to be demonstrated.

Male ddY mice (about 7 weeks old) were used (n=15 to 30), and provided with food and water ad libitum. As test substances, Ser-Trp-Met-His-Gln-Pro-His-Gln-Pro-Leu-Pro-Pro-Thr-Val-Met-Phe-Pro-Pro-Gln-Ser-Val-Leu (SEQ ID NO: 3) at 500 nmol/kg (1280 µg/kg body weight), or Met-His-Gln-Pro-His-Gln-Pro-Leu-Pro-Pro-Thr-Val-Met-Phe-Pro-Pro-Gln-Ser-Val-Leu (MHQPHQPLPPTVMFPPQSVL; SEQ ID NO: 16) at 500 nmol/kg body weight (1140 µg/kg body weight), or Leu-Gln-Ser-Trp-Met-His-Gln-Pro-His-Gln-Pro-Leu-Pro-Pro-Thr-Val-Met-Phe-Pro-Pro-Gln-Ser-Val-Leu (LQSWMHQPHQPLPPTVMFPPQSVL; SEQ ID NO: 4) at 500 nmol/kg body weight (1400 µg/kg body weight), or Ser-Trp-Met-His-Gln-Pro-His-Gln-Pro-Leu-Pro-Pro-Thr-Val-Met-Phe-Pro-Pro-Gln (SWMHQPHQPLPPTVMFPPQ; SEQ ID NO: 17) at 500 nmol/kg body weight (1150 µg/kg body weight) were used. The percentage of change in the spontaneous alternation behavior was determined as described in Example 4, and used as an indicator of the spontaneous alternation behavior. The measured values were expressed as mean ± standard error for each group. The significance of difference between the control group and the scopolamine control group was tested by Student's t-test. The significance of difference between the scopolamine control group and each peptide-administered group was tested by Student's t-test.

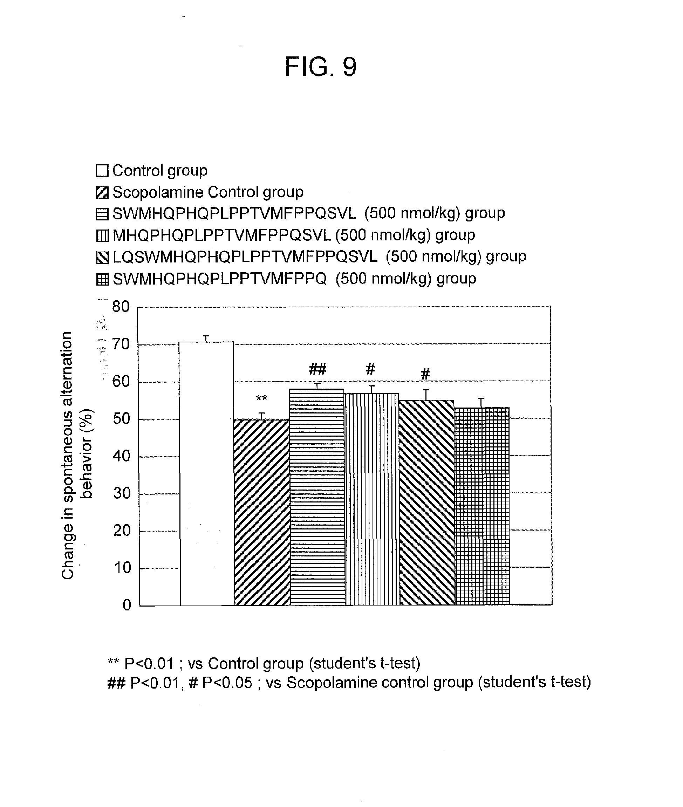

The results are shown in FIG. 9. SWMHQPHQPLPPTVMFPPQSVL (SEQ ID NO: 3) at 500 nmol/kg body weight (1280 µg/kg body weight), or MHQPHQPLPPTVMFPPQSVL (SEQ ID NO: 16) at 500 nmol/kg body weight (1140 µg/kg body weight), or LQSWMHQPHQPLPPTVMFPPQSVL (SEQ ID NO: 4) at 500 nmol/kg body weight (1400 µg/kg body weight) were shown to have amnesia-preventing actions. However, the significant difference could not be confirmed for SWMHQPHQPLPPTVMFPPQ (SEQ ID NO: 17) at 500nmol/kg body weight (1150 µg/kg body weight).

### INDUSTRIAL APPLICABILITY

According to the present invention, a method for preparing casein-derived peptides is provided. The casein-derived peptides mainly have a brain function-improving action. Such functional peptides can be easily and efficiently prepared by the method of the present invention. Also, a composition comprising the peptide, or a method of preparing a functional food is provided according to the present invention. Accordingly, the present invention is useful in the field of pharmaceuticals, food and drink products, health-promotion and other fields.

### ACCESSION NUMBER:

Accession Number FERM BP-6060 *(Lactobacillus helveticus* CM4 strain, deposited on August 15,1997)
Accession Number FERM BP-11271 *(Lactobacillus helveticus* CP3232 strain, deposited on August 4, 2010)

### SEQUENCE LISTING FREE TEXT

SEQ ID NOs: 5 to 8 and 10 to 17: Artificial proteins (functional peptides)
SEQ ID NO: 9: Artificial protein (synthetic peptide)

## Claims

1. A method for preparing a peptide consisting of the amino acid sequence shown in any of SEQ ID NOS: 1 to 4 or 16, comprising fermenting animal milk or milk protein using at least one lactic acid bacterium and/or a treated product thereof and collecting the peptide from the resultant fermented milk.

2. The method according to claim 1, wherein the lactic acid bacterium is at least one bacterium belonging to a genus selected from the group consisting of *Lactobacillus, Streptococcus, Bifidobacterium, Enterococcus, Leuconostoc, Lactococcus, Pediococcus,* and *Weissella.*

3. The method according to claim 1, wherein the lactic acid bacterium is at least one selected from the group consisting of *Lactobacillus helveticus, Lactobacillus delbrueckii* subsp. *bulgaricus, Lactobacillus acidophilus, Lactobacillus casei, Lactobacillus amylovorus, Lactobacillus gasseri, Lactobacillus paracasei, Lactobacillus zeae, Lactobacillus rhamnosus, Lactobacillus reuteri*, *Lactobacillus crispatus, Lactobacillus gallinarum, Lactobacillus brevis, Lactobacillus fermentum*, *Lactobacillus plantarum,* and *Lactobacillus johhsonii.*

4. The method according to claim 1 or 2, wherein the lactic acid bacterium is *Streptococcus thermophilus.*

5. The method according to claim 1 or 2, wherein the lactic acid bacterium is *Lactobacillus helveticus* CM4 strain (Accession Number: FERM BP-6060).

6. The method according to any one of claims 1 to 5, wherein the peptide is collected 3 to 96 hours after the start of the fermentation.

7. A method for preparing a composition comprising a peptide consisting of the amino acid sequence shown in any of SEQ ID NOS: 1 to 4 or 16, comprising fermenting animal milk or milk protein using at least one lactic acid bacterium and/or a treated product thereof and formulating the resultant fermented milk or the peptide consisting of the amino acid sequence shown in any of SEQ ID NOS: 1 to 4 or 16 collected from the fermented milk.

8. A composition comprising a peptide consisting of the amino acid sequence shown in any of SEQ ID NOS: 1 to 4 or 16.

9. A fermented milk composition, which is obtained by fermenting animal milk or milk protein using at least one lactic acid bacterium and/or a treated product thereof and comprises a peptide consisting of the amino acid sequence shown in any of SEQ ID NOS: 1 to 4 or 16.

10. The composition according to claim 9, wherein the composition comprises 0.1 µg/ml or more of the peptide.

11. A food or drink comprising the composition according to any one of claims 8 to 10.

12. A supplement comprising the composition according to any one of claims 8 to 10.

13. A method for preparing a functional food or drink, comprising fermenting animal milk or milk protein using at least one lactic acid bacterium and/or a treated product thereof, and incorporating the resultant fermented milk or a peptide consisting of the amino acid sequence shown in any of SEQ ID NOS: 1 to 4 or 16 obtained therefrom, into a food or drink.

14. Use of at least one lactic acid bacterium or a treated product thereof for producing a peptide consisting of the amino acid sequence shown in any of SEQ ID NOS: 1 to 4 or 16.


SEQUENCE LISTING

<110> Calpis Co., Ltd.

<120> Production method for casein-derived peptides by lactic acid
fermentation

<130> P104018EP

<140> 12802461.9
<141> 2012-06-12

<150> JP 2011-140737
<151> 2011-06-24

<160> 18

<170> PatentIn version 3.5

<210> 1
<211> 19
<212> PRT
<213> Artificial

<220>
<223> Functional peptide

<400> 1

Asn Ile Pro Pro Leu Thr Gln Thr Pro Val Val Val Pro Pro Phe Leu
1               5                   10                  15

Gln Pro Glu

<210> 2
<211> 21
<212> PRT
<213> Artificial

<220>
<223> Functional peptide

<400> 2

Asn Ile Pro Pro Leu Thr Gln Thr Pro Val Val Val Pro Pro Phe Leu
1               5                   10                  15

Gln Pro Glu Val Met
            20

<210> 3
<211> 22
<212> PRT
<213> Artificial

<220>
<223> Functional peptide

<400>  3

Ser Trp Met His Gln Pro His Gln Pro Leu Pro Pro Thr Val Met Phe
1               5                   10                  15

Pro Pro Gln Ser Val Leu
            20

<210>  4
<211>  24
<212>  PRT
<213>  Artificial

<220>
<223>  Functional peptide

<400>  4

Leu Gln Ser Trp Met His Gln Pro His Gln Pro Leu Pro Pro Thr Val
1               5                   10                  15

Met Phe Pro Pro Gln Ser Val Leu
            20

<210>  5
<211>  19
<212>  PRT
<213>  Artificial

<220>
<223>  functional peptide

<220>
<221>  misc_feature
<222>  (1)..(1)
<223>  Xaa = absent, or Ile or Asn-Ile

<220>
<221>  misc_feature
<222>  (19)..(19)
<223>  Xaa = absent or Val-Met

<400>  5

Xaa Pro Pro Leu Thr Gln Thr Pro Val Val Val Pro Pro Phe Leu Gln
1               5                   10                  15

Pro Glu Xaa

<210>  6
<211>  21
<212>  PRT
<213>  Artificial

<220>
<223>  functional peptide


<220>
<221>  misc_feature
<222>  (1)..(1)
<223>  Xaa = absent, or Ser-Trp or Leu-Gln-Ser-Trp

<400>  6

Xaa Met His Gln Pro His Gln Pro Leu Pro Pro Thr Val Met Phe Pro
1               5                   10                  15


Pro Gln Ser Val Leu
            20


<210>  7
<211>  18
<212>  PRT
<213>  Artificial

<220>
<223>  functional peptide

<400>  7

Ile Pro Pro Leu Thr Gln Thr Pro Val Val Val Pro Pro Phe Leu Gln
1               5                   10                  15


Pro Glu




<210>  8
<211>  18
<212>  PRT
<213>  Artificial

<220>
<223>  functional peptide

<400>  8

Asn Ile Pro Pro Leu Thr Gln Thr Pro Val Val Val Pro Pro Phe Leu
1               5                   10                  15


Gln Pro




<210>  9
<211>  10
<212>  PRT
<213>  Artificial

<220>
<223>  synthetic peptide

<400>  9

Thr Gln Thr Pro Val Val Val Pro Pro Phe
1               5                   10


<210>  10
<211>  17
<212>  PRT
<213>  Artificial

<220>
<223>  functional peptide

<400>  10

Pro Pro Leu Thr Gln Thr Pro Val Val Val Pro Pro Phe Leu Gln Pro
1               5                   10                  15


Glu


<210>  11
<211>  14
<212>  PRT
<213>  Artificial

<220>
<223>  functional peptide

<400>  11

Thr Gln Thr Pro Val Val Val Pro Pro Phe Leu Gln Pro Glu
1               5                   10


<210>  12
<211>  16
<212>  PRT
<213>  Artificial

<220>
<223>  functional peptide

<400>  12

Pro Leu Thr Gln Thr Pro Val Val Val Pro Pro Phe Leu Gln Pro Glu
1               5                   10                  15


<210>  13
<211>  15
<212>  PRT
<213>  Artificial

<220>
<223>  functional peptide

<400>  13

Leu Thr Gln Thr Pro Val Val Val Pro Pro Phe Leu Gln Pro Glu

1               5                   10                  15


<210>  14
<211>  11
<212>  PRT
<213>  Artificial

<220>
<223>  functional peptide

<400>  14

Pro Val Val Val Pro Pro Phe Leu Gln Pro Glu
1               5                   10


<210>  15
<211>  10
<212>  PRT
<213>  Artificial

<220>
<223>  functional peptide

<400>  15

Val Val Val Pro Pro Phe Leu Gln Pro Glu
1               5                   10


<210>  16
<211>  20
<212>  PRT
<213>  Artificial

<220>
<223>  functional peptide

<400>  16

Met His Gln Pro His Gln Pro Leu Pro Pro Thr Val Met Phe Pro Pro
1               5                   10                  15


Gln Ser Val Leu
            20


<210>  17
<211>  19
<212>  PRT
<213>  Artificial

<220>
<223>  functional peptide

<400>  17

Ser Trp Met His Gln Pro His Gln Pro Leu Pro Pro Thr Val Met Phe
1               5                   10                  15

Pro Pro Gln

<210> 18
<211> 5
<212> PRT
<213> Artificial Sequence

<220>
<223> Synthetic peptide

<220>
<221> MISC_FEATURE
<222> (1)..(1)
<223> Xaa may be Leu, Ile, Met, Phe or Trp

<400> 18

Xaa Pro Leu Pro Arg
1               5